# EUROPEAN PATENT APPLICATION

(11) **EP 4 632 077 A1**
(43) Date of publication of application: **15.10.2025**
(21) Application number: 23901144.8
(22) Date of filing: 08.12.2023
(51) Int. Cl.: C12Q 1/6869, C12Q 1/6886

(54) **MULTIBODY FULL-LENGTH SEQUENCING ANALYSIS METHOD FOR SINGLE CELL USING MULTI-COMBINATION ASSEMBLY REACTION OF DNA FRAGMENTS**

(30) Priority: 08.12.2022 KR 20220170207
(71) Applicant: EYEONCELL CO., LTD., Gwangju 61005 (KR)
(72) Inventor: PARK, Jihwan, Gwangju 61005 (KR); AN, Hyunsu, Gwangju 61005 (KR); CHOI, Sin Young, Gwangju 61005 (KR)
(74) Representative: Synergy IP Group AG
(86) International application number: PCT/KR2023/020167
(87) International publication number: WO 2024/123127

(57) **Abstract**

The present invention relates to a single cell multibody full-length sequencing analysis technique. The present invention is a development study to overcome the limitations of research on low quality and cell heterogeneity of multiplex full-length sequencing of a single cell, wherein an assembly method that allows the combination of multiple DNA fragments is used to greatly improve the efficiency and error rate of full-length sequencing, and the full-length sequencing of a single cell multibody is completed and thus can be greatly used for gene isoform expression level analysis, mutation detection, or cancer cell-specific multibody analysis.

## Description

### [Technical Field]

The present disclosure relates to multiome full-length sequencing analysis method for single cell using multi-combination assembly reaction of DNA fragments.

### [Background Art]

The ability to accurately and rapidly sequence the genome has enabled innovative biology and medicine. Research on complex genomes, and particularly studies on the genetic basis of diseases in humans, involves large-scale genetic analysis. Genetic analysis at the whole-genome level is not only financially expensive, but also time- and labor-consuming. These costs increase when using protocols that include the analysis of separate individual DNA samples. Sequencing of polymorphic regions in genomes associated with disease greatly contributes to understanding diseases such as cancer and drug development, and leads to fulfilling pharmacogenomic tasks aimed at identifying genes and functional polymorphisms related to variability in drug response.

After the completion of the Human Genome Project, there has been a dramatic advancement in next-generation sequencing (NGS) technology over the past ten years. Second-generation NGS technologies such as Illumina or IonTorrent are very powerful, but they can only sequence short reads of 100 to 500 bp in length, so the length of analyzable molecules is limited. Therefore, second-generation technologies have limitations in understanding complex genomic structures such as repetitive sequences and copy number variations (CNVs). To improve this, third-generation long-read sequencing methods have been developed. However, there are limitations in preparing long-read single-molecule sequencing libraries for third-generation methods, and especially for long-read sequencing, high-cost problems, and limitations in the resolution of cellular heterogeneity research have emerged. Accordingly, there is a growing need for more efficient and improved sequencing.

Therefore, the present invention is a development study to overcome the low quality and high cost of multiome full-length sequencing, including transcriptomes of single cells, and the limitations of cellular heterogeneity studies. By using an assembly method that allows the combination of multiple DNA fragments, it significantly improves the efficiency and error rate of full-length sequencing, and enables multiome full-length sequencing of single cells. It is expected to be widely used for expression analysis of gene isoforms, mutation detection, or cancer cell-specific multiome analysis.

### [Disclosure]

### [Technical Problem]

The present inventors have diligently conducted research to overcome the low quality of multiome full-length sequencing of single cells and the limitations in studying cellular heterogeneity. As a result, by using an assembly method that allows the combination of multiple DNA fragments, they significantly improved the efficiency and error rate of full-length sequencing, and completed multiome full-length sequencing of single cells, thereby completing the present invention.

Accordingly, the object of the present invention is to provide a method for full-length multiome sequencing analysis of a single cell, which provides a sequencing analysis method comprising the following:
(a) a step of attaching a multi-ligation assembly adapter of DNA fragments to a multiome library prepared from a single cell;
(b) a step of selectively circularizing DNA molecules in the library through a multi-ligation assembly reaction of DNA fragments to select DNA molecules within the library; and
(c) a step of removing DNA fragments other than the circular DNA using an exonuclease.

However, objects to be achieved by the present disclosure are not limited to the objects mentioned above, and other objects not mentioned above may be clearly understood by those skilled in the art from the following description.

### [Technical Solution]

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

According to one aspect of the present invention, a method for full-length multiome sequencing analysis of a single cell is provided, comprising the following steps: (a) attaching a multi-ligation assembly adapter of DNA fragments to a multiome library prepared from a single cell; (b) selectively circularizing DNA molecules in the library through a multi-ligation assembly reaction of DNA fragments to select DNA molecules within the library; and (c) removing DNA fragments other than the circular DNA using an exonuclease.

The method may further comprise, after step (c), (d) a step of linearizing the circular DNA in the library.

In the method, the multiome may be a transcriptome, spatial transcriptome, genome, spatial genome, proteome, epigenome, or spatial epigenome, and the multiome library may comprise different adapters (forward and reverse adapters) ligated to the 3' and 5' ends, respectively, and step (a) may be a process in which two different adapters are assembled to form circular DNA.

When step (d) is included in the method, the linearization in step (d) may be performed using a CRISPR/Cas nuclease, restriction enzyme, or transcription activator-like effector nuclease (TALEN), and the CRISPR/Cas nucleases may be any one selected from the group consisting of CRISPR/Cas9, CRISPR/Cas12, CRISPR/Cas13, and CRISPR/Cas14.

The inventors of the present invention have diligently studied to overcome the limitations of low quality in single-cell multiome full-length sequencing and the challenges in cell heterogeneity research. As a result, by utilizing a multi-ligation assembly reaction of DNA fragments, they have significantly improved the efficiency and error rate of full-length sequencing, enabled full-length sequencing of single-cell multiome, and, in particular, achieved the selective sequencing of only desired target DNA molecules.

According to the present invention, "Ouroboros" refers to an ancient symbol in the form of a snake eating its own tail, and represents a technology for selectively removing or enriching various DNA targets using an assembly method that allows the joining of multiple DNA fragments. By utilizing an assembly reaction that permits the ligation of multiple DNA fragments, the technology enables selective targeting of DNA with cell barcodes. Using this selection technology, it is possible to perform target enrichment for detecting cancer gene mutations, mitochondrial genome-derived cDNA, and specific cell populations. Additionally, depletion technology can be applied to remove ribosomal and mitochondrial genome-derived cDNA.

In the present specification, the term "biological sample" refers to any substance, biological fluid, tissue, or cell obtained from or derived from an organism. Examples include, but are not limited to, whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid.

In the present specification, the term "transcriptome" refers to the total set of all expressed RNA. Related terms include genome, epigenome, spatial transcriptome, proteome, connectome, and multiome, which encompasses all of these.

In the present specification, the term "library" refers to a collection of DNA molecules of sufficient quantity to include all individual genes present within a specific organism.

According to the present invention, the multi-fragment assembly reaction of DNA fragments, also referred to as multi-DNA fragment assembly, refers to a molecular assembly method that allows the joining of multiple DNA fragments in a single isothermal reaction. Specifically, it may be a Gibson assembly reaction, but is not limited thereto.

In the present specification, the term "exonuclease" refers to an enzyme that acts by cleaving nucleotides one at a time from the ends of a polynucleotide. A hydrolysis reaction occurs that breaks the phosphodiester bonds at either the 3' or 5' end.

According to specific embodiments of the present invention, the multiome may be a transcriptome, genome, spatial transcriptome, epigenome, or proteome.

According to specific embodiments of the present invention, the single cell is used to construct a single-cell transcriptome and genome library based on one or more techniques selected from the group consisting of: 10X Genomics, Drop-Seq, SPLiT-Seq, Sci-Seq, Microwell-Seq, SMART-Seq, Fluidigm C1 single-cell technology, BGI DNBelab C4 single-cell technology, CITE-Seq, CEL-Seq, scifi-RNA-Seq, Sci-Seq, Visium, Slide-Seq, Seq-Scope, and Stereo-Seq technologies.

In the present specification, the term "10X Genomics single-cell sequencing" refers to a single-cell RNA sequencing (RNA-sequencing) technology. It uses microfluidic partitioning to capture individual cells and prepare barcoded next-generation sequencing (NGS) cDNA libraries, allowing transcriptome analysis on a per-cell basis.

According to specific embodiments of the present invention, the transcriptome library is constructed such that a Read1 (R1) primer and a cell barcode are attached to the 3' end of the RNA sequence, and a template-switching oligo (TSO) primer is attached to the 5' end.

According to the present invention, a template-switching oligo (TSO) refers to an oligonucleotide that hybridizes to the untemplated C nucleotides added by the reverse transcriptase during reverse transcription. The TSO adds a common 5' sequence to the full-length cDNA, which is used for downstream cDNA amplification.

According to specific embodiments of the present invention, the multiome library is constructed such that a forward adaptor sequence is attached to the 3' end and a reverse adaptor sequence is attached to the 5' end.

The "primer" of the present invention is a fragment that recognizes the target gene sequence and includes a pair of forward and reverse primers. Preferably, the primer pair provides analytical results with specificity and sensitivity. When the nucleotide sequence of the primer is mismatched with the non-target sequences present in the sample, and the primer amplifies only the target sequence containing complementary primer binding sites without causing non-specific amplification, high specificity can be achieved.

According to specific embodiments of the present invention, the (b) step involves the formation of a circular DNA by assembling the forward adapter and reverse adapter into an assembly adapter.

According to specific embodiments of the present invention, the DNA to be linearized in the step is the full-length multiome library of a single cell.

According to specific embodiments of the present invention, the linearization step involves using CRISPR/Cas nucleases, restriction enzymes, or TALEN (Transcription Activator-Like Effector Nucleases) to linearize the DNA.

According to specific embodiments of the present invention, the CRISPR/Cas nucleases are selected from the group consisting of CRISPR/Cas9, CRISPR/Cas12, CRISPR/Cas13, and CRISPR/Cas14.

In this specification, the term "CRISPR (Clustered regularly interspaced short palindromic repeats)-Cas" refers to a gene editing technology originating from the adaptive immune system of microorganisms. It allows for simpler and more efficient genetic manipulation by precisely cutting target DNA and then allowing the DNA to naturally repair itself. This system consists of a guide RNA (guide RNA; gRNA) and a Cas nuclease, and is also known as RNA-guided engineered nucleases (RGENs) technology. The gRNA consists of crRNA, which binds complementarily to the target sequence, and tracrRNA, which is required for Cas binding. The gRNA serves to guide Cas to the target region and binds complementarily to the target DNA sequence. The target DNA sequence must contain a PAM (Protospacer adjacent motif) sequence at the 3' end. When the gRNA forms a complex with the Cas nuclease and specifically binds to the target sequence, the Cas nuclease recognizes the PAM sequence and induces a double-strand break (DSB) in the 3 bp upstream region of the PAM sequence.

According to the present invention, the Cas is selected from the group consisting of Cas9, Cas12, Cas13, and Cas14, which are components of the CRISPR/Cas complex. Specifically, it may be Cas9, but it is not limited to this.

In this specification, the term "Cas9 (CRISPR associated protein 9)" refers to a 160 kDa protein that plays a crucial role in the immune defense of certain bacteria against DNA viruses and plasmids. It is widely used in genetic engineering applications, and its main function is to cut DNA, thereby modifying the cell's genome.

In this specification, the term "Cas12" refers to an RNA-guided endonuclease that forms part of the CRISPR system in certain bacteria. In addition to cutting the target gene according to the recognition of the protospacer, Cas12 also induces collateral cutting of non-targeted single-stranded DNA (ssDNA). Cas12 is used in the development of various portable diagnostic technologies, leveraging its collateral cutting function. When the Cas12a crRNA complex binds to the target double-strand DNA, it causes approximately 1,250 collateral cuts per second in single-stranded DNA, making Cas12a an enzyme capable of simultaneous nucleic acid detection and signal amplification. Cas12 specifically recognizes sequences with a 5'-(T)TTN-3' PAM sequence in double-stranded structures. The commonly used Cas12 enzymes are Cas12a and Cas12b. In the case where the target is single-stranded DNA, the PAM sequence does not need to match 100% but still induces catalytic cleavage even with variations.

In this specification, the term "Cas13" refers to an RNA-guided RNA endonuclease, which does not cut DNA but only cleaves single-stranded RNA. The Cas13 group forms a ribonucleoprotein complex with gRNA that consists of a 28-30 bp spacer capable of recognizing the target gene. The Cas13 protein acts as an RNA-degrading enzyme, unlike Cas12, which cleaves DNA. Cas13 induces collateral RNA cleavage instead of DNA cleavage. Among the Cas13 group, Cas13a, formerly known as C2C2, was the first to be used for nucleic acid detection. Cas13a cleaves single-stranded RNA targets with a protospacer matching the crRNA. Once bound to single-stranded RNA, the Cas13a protein acts as a non-specific RNA-degrading enzyme, degrading surrounding RNA even if it is not the target RNA. It has been reported that one target recognition can induce at least 10^4 collateral cleavages. Cas13 cleaves both the target gene and non-specific surrounding RNA sequences. Since Cas13 cleaves around the complementary protospacer, not directly at the protospacer, the protospacer remains intact, which allows multiple cleavages and can result in an amplification effect, making it useful for sensitive detection. Cas13 does not require a PAM sequence but needs a protospacer flanking site (PFS), where the base guanine is adjacent to the protospacer. The most widely used Cas13 proteins in biotechnology and diagnostics are Cas13a and Cas13b, with Cas13b known to be more stable for cell gene manipulation.

According to another aspect of the present invention, a method for sequencing analysis after selective removal of DNA molecules from a full-length multiome library of a single cell is provided, comprising the following steps: (a) attaching a multi-ligation assembly adapter of DNA fragments to a multiome library prepared from a single cell; (b) generating circular DNA within the library through a multi-ligation assembly reaction of DNA fragments; (c) selectively linearizing circular DNA containing target sequences to be removed using a CRISPR/Cas nuclease; and (d) removing linear DNA, which is not circular DNA, using an exonuclease.

This method may further comprise, after step (d), (e) linearizing the circular DNA in the library.

In the method, the CRISPR/Cas nucleases may be any one selected from the group consisting of CRISPR/Cas9, CRISPR/Cas12, CRISPR/Cas13, and CRISPR/Cas14, and the CRISPR/Cas nuclease may be applied only to circular DNA. The linear DNA in step (d) may be a molecule generated by degradation of selected circular DNA recognized by the CRISPR/Cas nuclease.

In the present specification, the term "CRISPR/Cas9" refers to a powerful third-generation gene editing tool (Genome Editing Tool) that is more economical and efficient than the first-generation ZFN (Zinc Finger Nuclease) and the second-generation TALEN (Transcription Activator-Like Effector Nuclease). The CRISPR-Cas9 system consists of guide RNA that accurately recognizes and binds to target DNA, and Cas9 nuclease that cuts the target DNA, inducing double-strand breaks, which are repaired, allowing gene manipulation.

In a specific embodiment of the invention, the CRISPR/Cas nuclease is selected from the group consisting of CRISPR/Cas9, CRISPR/Cas12, CRISPR/Cas13, and CRISPR/Cas 14.

According to the invention, the cleavage of a DNA molecule refers to a reaction that cuts one of the covalent sugar-phosphate linkages between nucleotides that make up the sugar-phosphate backbone of DNA. This reaction is catalyzed by enzymes, chemicals, or radiation.

In a specific embodiment of the invention, the CRISPR/Cas nuclease is applied only to circular DNA.

In another specific embodiment of the invention, the linear DNA in the step of removing linear DNA is a selected DNA molecule recognized by the CRISPR/Cas nuclease.

In a further specific embodiment of the invention, the DNA in step (e) is a full-length multiome library of single cells.

According to another aspect of the invention, a method for sequencing analysis after selection of DNA molecules from a full-length multiome library of a single cell is provided, comprising the following steps: (a) preparing a multiome library from a single cell and attaching a multi-ligation assembly adapter of DNA fragments to the library; (b) generating circular DNA within the library through a multi-ligation assembly reaction of DNA fragments; (c) removing DNA fragments that are not circular DNA using an exonuclease; and (d) selectively converting circular DNA containing target sequences to be selected into linear DNA using a CRISPR/Cas nuclease.

This method may further comprise, after step (d), (e) amplifying the linear DNA in the library.

In the method, the CRISPR/Cas nuclease may be any one selected from the group consisting of CRISPR/Cas9, CRISPR/Cas12, CRISPR/Cas13, and CRISPR/Cas14, and the linear DNA in step (d) may be a molecule generated by degradation of selected circular DNA recognized by the CRISPR/Cas nuclease.

When step (e) is included in the method, the step may further comprise a ligation step of a Y-adaptor or a hairpin adaptor.

According to the invention, Y-adaptor ligation refers to the method of attaching synthetic oligonucleotides with known nucleotide sequences to both ends or the ends of DNA fragments.

In a specific embodiment of the invention, the multiome may be a full-length cDNA transcriptome, spatial transcriptome, genome, spatial genome, proteome, epigenome, or spatial epigenome library derived from a single cell.

According to the invention, single-cell sequencing analysis technology can analyze the proteome, transcriptome, genome, spatial transcriptome, or epigenome of a single cell. It allows for the simultaneous analysis of various cells present within a tissue and provides accurate information regarding the type, lineage, disease, and mutations of individual cells. Single-cell sequencing technology can be effectively used in fields such as cancer tumor heterogeneity research, immunology, or developmental biology. Specifically, in immunology research, it enables a comprehensive analysis of the immune system, characterization of immune cell populations (clonal analysis), and reconstruction of immune cell development pathways. Additionally, through cancer tumor heterogeneity research, it allows for the analysis of cancer stem cells or circulating tumor cells (CTCs).

According to the invention, single-cell analysis refers to a technology that analyzes genomics, transcriptomics, spatial transcriptomics, epigenomics, or proteomics at the single-cell level, as opposed to methods that analyze multiple cells simultaneously. A single cell refers to one cell isolated from a cell population, and since cell heterogeneity exists even among cells derived from the same origin, research at the single-cell level is crucial for accurate analysis. This is especially emphasized in diseases that require the analysis of inherently heterogeneous cell populations, such as stem cell research or cancer, where molecular markers must be investigated at the single-cell level rather than at the average level of a population of cells. With the advancement of single-cell analysis technology, there is growing potential to construct a systematic Reference Map of all human cells. Several countries are conducting projects that analyze various disease tissues using single-cell analysis, and these studies are actively contributing to diagnostics, healthcare, and drug development. Traditional bulk analysis methods, which aggregate data from multiple cells, have been instrumental in accumulating information related to specific omics areas. However, they only provide average values for each omic, making it difficult to accurately identify signals directly related to diseases. In contrast, single-cell multi-omics analysis can provide a more complete understanding of each cell by examining each omic individually, allowing for the more accurate elucidation of the complex interaction networks that govern cellular functions. Even if cells are derived from the same parent cell, they can generate different cell types, and tissues are composed of these heterogeneous cell populations. Therefore, identifying and characterizing individual cell specificity through single-cell multi-omics analysis is crucial for accurate disease diagnosis and treatment. Furthermore, single-cell multi-omics analysis integrates and analyzes multi-layered omic information about specific cells, enabling the rapid extraction of meaningful insights.

According to the invention, multi-omics analysis is an advanced research technique that measures the comprehensive changes in various biomolecules within cells and integrates this data for analysis. It is particularly useful in fundamental life sciences and clinical research for interpreting gene functions, understanding disease mechanisms, and more. With the introduction of next-generation sequencing (NGS) techniques, it has become possible to generate large-scale multi-omics data at low cost, leading to the widespread availability of multi-omics big data.

According to the invention, multi-omics includes genomics, epigenomics, transcriptomics, spatial transcriptomics, or proteomics, which are related to information transmission and regulation within organisms. It also includes metabolites such as metabolomics and lipidomics, which are cellular metabolic products.

According to the invention, genomics research refers to the omics field that studies the genetic composition of individuals using next-generation sequencing (NGS) techniques. It includes whole-genome sequencing (WGS) to measure the entire genome, whole-exome sequencing (WES) to analyze protein-coding regions, and single nucleotide polymorphism (SNP) genotyping studies. The genomic information generated by these techniques is used in genome-wide association studies (GWAS) to identify causal variants that contribute to diseases. Based on this, genomics research in genomic medicine is actively exploring the prediction of genetic risk for diseases and drug response. Additionally, this field is applied in various areas of life sciences, such as population genetics, evolutionary biology, and synthetic biology.

According to the invention, epigenomics research refers to the field studying the epigenetic modifications of the genome, which involves interpreting phenotypic traits influenced by environmental factors. It primarily investigates the changes in DNA methylation and histone modifications, as well as their association with genetic regulation. This research allows for the interpretation of phenotypic diversity (phenotypic plasticity) that cannot be explained solely by genetic composition. Experimental techniques such as bisulfite sequencing to measure DNA methylation, chromatin immunoprecipitation (ChIP)-sequencing to identify histone modification marking regions, and DNase/ATAC-seq for analyzing open/closed chromatin regions are used in this field. Recently, research in epitranscriptomics, focusing on modifications of the transcriptome like m6A, has been actively conducted, shedding light on the causes of various biological phenomena such as cell differentiation, development, and tumor formation.

According to the invention, transcriptomics research refers to the omics field that comprehensively studies gene expression, including not only coding genes involved in protein synthesis but also non-coding RNAs like microRNAs that do not participate in coding. It gained significant attention in the early 2000s with the introduction of microarray technology and has since been largely replaced by RNA sequencing (RNA-seq) following the advent of next-generation sequencing (NGS) technologies in 2010. Recently, with the introduction of single-cell transcriptomics, it has become possible to analyze gene expression in different cell types within tissues, making it a valuable tool for studying disease mechanisms. Transcriptomics research is widely used in life sciences, particularly in areas such as cell differentiation, stem cells, tumor formation, gene regulation, and biomarker discovery. It is also applied in expression profiling, alternative splicing analysis, RNA editing analysis, and more.

According to the invention, spatial transcriptome research refers to a technique that simultaneously observes the positional information of each cell and its gene expression pattern in tissue sections, enabling the study of cell distribution and cell-to-cell interactions within the actual tissue. High-resolution technologies that can distinguish from tens of cells to compartments within individual cells have been developed, and this approach is particularly useful in understanding the microenvironment of cancer tissues.

According to the invention, proteomics research refers to the omics field that analyzes the expression or modification of proteins, which directly mediate physiological processes in organisms. It is a key aspect of functional genomics, which has gained significant attention since the Human Genome Project. This field allows the analysis of post-translational modifications and protein isoforms that cannot be identified through genomic or transcriptomic analyses, as well as the discovery of various biomarkers used in clinical settings. Due to the inherent complexity of proteins, proteomics requires a variety of analytical techniques, including antibody-based affinity proteomics and mass spectrometry-based shotgun proteomics.

According to the invention, bead-based single-cell transcriptomics technology is one of the most commonly used techniques in single-cell sequencing. This droplet-based technology revolves around beads that contain a barcode. The principle of bead-based single-cell transcriptomics technology is as follows: First, a single cell is paired with a bead. Second, a barcode, derived from the bead, is attached to the DNA or RNA extracted from the cell. Third, after amplification and sequencing, the barcode is identified, allowing the analysis of the characteristics of individual cells. An example of the use of single-cell transcriptomics technology is the human cell atlas (Tabula Sapiens), in which the transcriptomes of approximately one million cells from 24 tissues and organs were analyzed.

According to the invention, the multi-ligation assembly reaction of DNA fragments is distinguished from existing technologies. While the process still uses the conventional circular DNA ligation, unlike traditional methods, the invention employs a more complex circularization process using a Uracil-containing primer in a hemi-nested gene-specific PCR, USERII enzyme, and T4 DNA ligase. This design is intended to address the reduced efficiency of PCR reactions with circular DNA, incorporating a step where the circular DNA is linearized using the CRISPR/Cas9 endonuclease before the PCR process. In contrast, existing methods do not include this linearization step and proceed directly with PCR. Specifically, in existing single-cell RNA-sequencing libraries, cell-barcode-containing molecules are selected using a biotin-tagged R1 primer. These molecules are then captured using streptavidin beads that bind to the biotin tag. However, this biotin-based selection method is significantly influenced by the size of the molecules, and when the DNA length exceeds 1 or 2 kb (the average length of human mRNA is approximately 2.2 kb), the selection efficiency dramatically decreases. Additionally, molecules with two cell barcodes (i.e., molecules with barcodes at both ends) are also selected, which can create issues as these molecules, despite being smaller and containing two biotin tags, are more efficiently captured, leading to potential problems. In contrast, the method of selecting cell-barcode-containing molecules using multi-ligation of DNA fragments in the invention has a significant advantage. It is less affected by the size of the DNA molecules and can effectively select molecules with a single barcode at one end, eliminating the issues of selecting molecules with two barcodes at both ends.

In addition, the conventional method for selecting cell-barcode-containing molecules in a single-cell RNA-sequencing library uses asymmetric PCR to linearly amplify molecules with cell barcodes, allowing the selective identification of these molecules. However, a significant issue arises with this approach: molecules with a cell barcode at only one end are amplified linearly, whereas atypical molecules with barcodes at both ends undergo exponential amplification. As a result, the number of such atypical molecules increases disproportionately, creating a problem with an excess of these undesirable molecules. In contrast, the method used in the present invention, which employs multi-ligation of DNA fragments for selecting cell-barcode-containing molecules, has a key advantage. It selectively identifies molecules with a single cell barcode at one end and effectively eliminates atypical molecules that either have barcodes at both ends or no barcodes at all. This method resolves the issue of unwanted amplification of malformed molecules, ensuring more accurate selection of molecules with the intended characteristics.

According to the present invention, the multi-ligation assembly reaction for selective DNA molecule removal is distinct from existing technologies. Specifically, the conventional technique for negative enrichment in single-cell RNA-seq libraries uses the CRISPR/Cas9 system to remove unwanted DNA (such as ribosomal RNA-derived cDNA) from the library. This method involves using 58 sgRNAs targeting specific sequences, and any linear DNA molecules containing one of these target sequences are recognized by the CRISPR/Cas9 system and cleaved. The resulting fragmented linear DNA molecules cannot properly bind the primers necessary for PCR amplification, and as a result, they do not undergo amplification in subsequent PCR steps. In contrast, the method of selective DNA molecule removal in the present invention applies the CRISPR/Cas9 system to circular DNA rather than linear DNA. When circular DNA is recognized and cleaved by the CRISPR/Cas9 system, it is converted into linear DNA, which is then completely removed from the library by exonuclease treatment. This approach avoids the potential issue in the conventional method where fragments of unwanted molecules (such as cleaved linear DNA) may remain in the PCR process, causing interference. By using exonuclease treatment to entirely remove unwanted DNA molecules before PCR amplification, the present invention ensures that the library amplification process is more accurate and free from such interference, offering a significant technical advantage over the conventional approach.

According to the present invention, the multi-ligation assembly reaction for selective cDNA target selection distinguishes itself from existing technologies. Specifically, the targeted selection technology using the Targeted Gene Expression Panel provided by 10X Genomics employs a method called hybridization capture. In this method, the single-cell RNA-Seq library is first separated into single-stranded molecules, which are then hybridized with biotin-labeled RNA/DNA probes. The biotin is attached to the target DNA molecules, and streptavidin beads are used to selectively capture the target DNA molecules. The hybridization capture-based targeted selection strategy relies on the binding of target DNA molecules to RNA/DNA probes, which requires complex experimental conditions to ensure that the target DNA molecules are separated into single strands and can effectively bind to the probes. Optimizing these conditions is challenging, as the diverse secondary structures that single-stranded target DNAs may adopt can hinder their binding to the RNA/DNA probes. Additionally, a complicated capture probe design algorithm is required to predict and prevent the formation of secondary structures that could arise from interactions between the thousands or tens of thousands of RNA/DNA probes. A further limitation of the hybridization capture method is that as the length of the selected molecules increases, the efficiency of the selection process decreases, making it difficult to select full-length cDNA. In contrast, the targeted selection strategy of the present invention uses the CRISPR/Cas9 system, which can precisely recognize the target sequences of DNA molecules. Even with thousands or tens of thousands of target DNA sequences, the sgRNAs that recognize these sequences form a stable complex with the CRISPR/Cas9 protein, creating an RNP (ribonucleoprotein) structure. This physical separation allows each sgRNA to independently search for its respective target DNA sequence without interference from the others. Unlike the hybridization capture method, which requires the RNA-Seq library to be processed into single-stranded form under complex conditions, the method in the present invention allows for the selection process to occur on double-stranded (dsDNA) molecules, making the selection process much simpler and more efficient.

Furthermore, the prior technology using the CRISPR/Cas9 system to select long DNA molecules relies on the principle that DNA molecules cleaved by CRISPR/Cas9 are marked with 5'-phosphate. In this method, first, the 5'-phosphate groups at the ends of linear DNA molecules are removed. Then, the CRISPR/Cas9 system is used to cut the region surrounding the target DNA, marking the cleaved target DNA region with 5'-phosphate. Since 5'-phosphate is required for DNA ligation, this feature allows for the attachment of sequencing adaptors exclusively to DNA molecules cleaved by CRISPR/Cas9. As a result, only the CRISPR/Cas9 cleaved DNA molecules are analyzed using long-read sequencing technology. This prior technology, however, encounters a problem where DNA is inevitably physically fragmented during sample processing (mechanical shearing), and the resulting fragments are randomly marked with 5'-phosphate at their cleavage sites. This random 5'-phosphate tagging results in a low efficiency in selecting the target DNA. In contrast, the selective DNA target selection method in the present invention employs a multi-ligation assembly reaction, where the unselected molecules are left in their circular DNA form, while only the selected target DNA molecules are converted into linear DNA for amplification. As a result, there is no need to remove or label the 5'-phosphate groups. Additionally, when purifying the circular DNA, exonuclease is used to degrade the linear DNA, preventing any adapter from accidentally attaching to the linear DNA that was intended to be removed. If necessary, the use of alkaline phosphatase, similar to the prior technology, can be incorporated to remove unwanted 5'-phosphates, ensuring that adapters are only attached to the CRISPR/Cas9-cleaved target DNA. This improvement reduces the need for complex processes while increasing the efficiency of target DNA selection.

The prior technology using the CRISPR/Cas9 system for selecting long DNA molecules also relies on the principle that CRISPR/Cas9 RNP (protein + RNA complex) strongly binds to the target DNA. In this method, DNA bound by CRISPR/Cas9 RNP is protected from degradation by exonuclease, similar to being "covered by a cap." As a result, the CRISPR/Cas9 system cuts the ends of the target DNA region, and only the DNA molecules protected by the CRISPR/Cas9 RNP are selected through exonuclease treatment. In contrast, the selective DNA target selection method in the present invention uses a multi-ligation assembly reaction, where unselected molecules are left in their circular DNA form, and only the selected target DNA is converted into linear DNA for amplification. Therefore, there is no need to select the molecules protected by CRISPR/Cas9 RNP using exonuclease. This distinction highlights that the technology used in the present invention, while also utilizing CRISPR/Cas9, is fundamentally different from the prior art.

Therefore, by efficiently performing sequencing analysis of single-cell multi-genomic full-length sequences through the present invention, it becomes possible not only to analyze gene expression but also to effectively identify gene isoforms and analyze their expression levels. Additionally, gene mutations can be effectively detected. By accurately distinguishing gene isoforms at the single-cell level, it becomes possible to propose new biomarkers for various types of cancer, which have previously been difficult to diagnose accurately. Examples of gene isoforms that are differentially expressed depending on cancer types are well summarized in the article "Splice variants as cancer biomarkers, Clinical Biochemistry, 2004." Currently, targeted cancer therapies are selected by first detecting genes that are highly expressed or mutated in cancer tissues and then selecting therapies that target these genes. However, cancer tissues are composed of various cancer cell clones, each containing different mutations. Therefore, the conventional approach of analyzing the entire cancer tissue to find target genes may have difficulty detecting the minority of cancer cell clones with different mutations. As a result, even when targeted therapy for mutations is performed, a minority of cancer cells with different mutations remain, leading to cancer recurrence. Thus, by analyzing cancer tissues at the single-cell level using the present invention, it will be possible to detect the minority of cancer cell clones with different mutations. With this information, targeted cancer therapies can be prescribed based on combinations that address the different mutations, potentially reducing the risk of recurrence.

According to another aspect of the invention, the invention provides a single-cell full-length multiome sequencing analysis kit utilizing the method for full-length multiome sequencing analysis of a single cell as described above.

According to another aspect of the invention, the invention provides a single-cell level gene isoform diagnostic marker analysis kit utilizing the method for full-length multiome sequencing analysis of a single cell as described above.

According to another aspect of the invention, the invention provides a single-cell mutation-based cancer targeted therapy candidate analysis kit utilizing the method for full-length multiome sequencing analysis of a single cell as described above.

### [Advantageous Effects]

The features and advantages of the present invention can be summarized as follows:
(a) The present invention provides a multiome full-length sequencing analysis technology for single cells.
(b) The present invention is a development study to overcome the low quality of multiome full-length sequencing of single cells and the limitations in cellular heterogeneity research. By using an assembly method that allows the combination of multiple DNA fragments, it significantly improves the efficiency and error rate of full-length sequencing, and by completing multiome full-length sequencing of single cells, it can be widely applied to gene expression analysis, gene isoform analysis, mutation detection, or cancer cell-specific multiome analysis. Furthermore, it can also be used for disease diagnosis based on gene isoforms and for mutation-based targeted cancer therapy.

### [Description of Drawings]

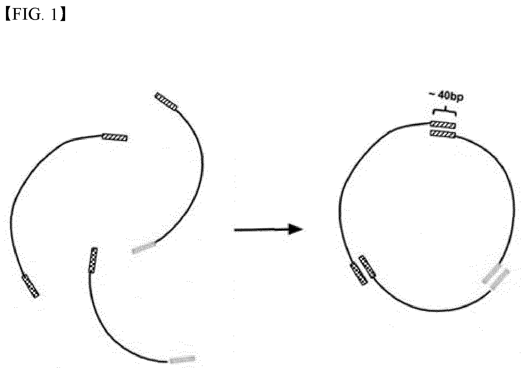

FIG. 1 shows a schematic diagram of the multi-combination assembly and replication method of DNA fragments.

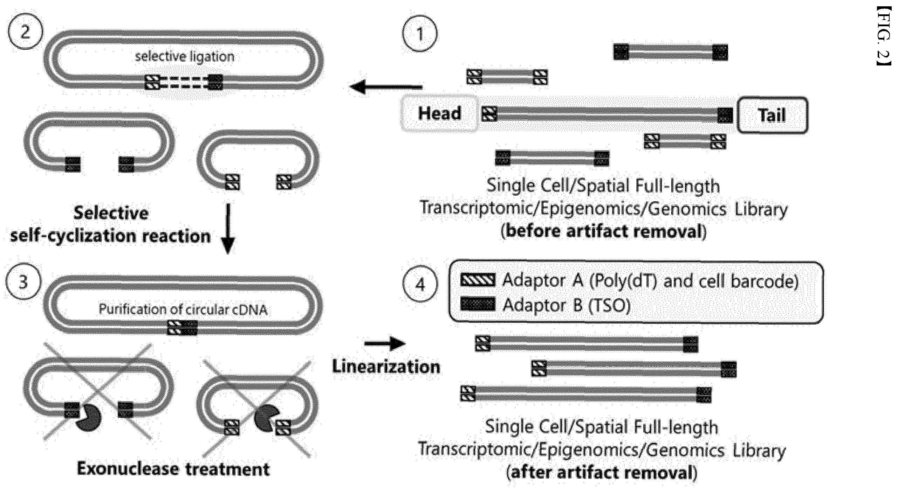

FIG. 2 shows the operating principle of the DNA selection method using the multi-combination assembly and replication method of DNA fragments.
FIG. 3 shows the results of selecting DNA molecules with cell barcodes using the multi-combination assembly reaction of DNA fragments.
FIG. 4 shows the results of selecting DNA molecules with cell barcodes using the multi-combination assembly reaction of DNA fragments from single cells of mouse heart.

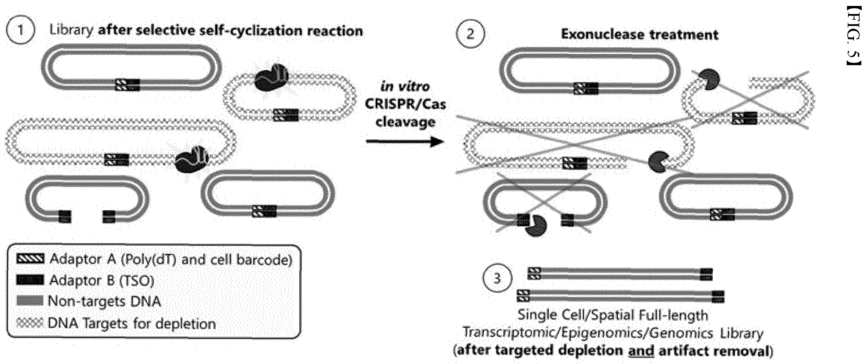

FIG. 5 shows the operating principle of selective DNA molecule removal using the multi-combination assembly reaction of DNA fragments.
FIG. 6 shows an example in which the selective DNA molecule removal method using the multi-combination assembly reaction of DNA fragments is applied.

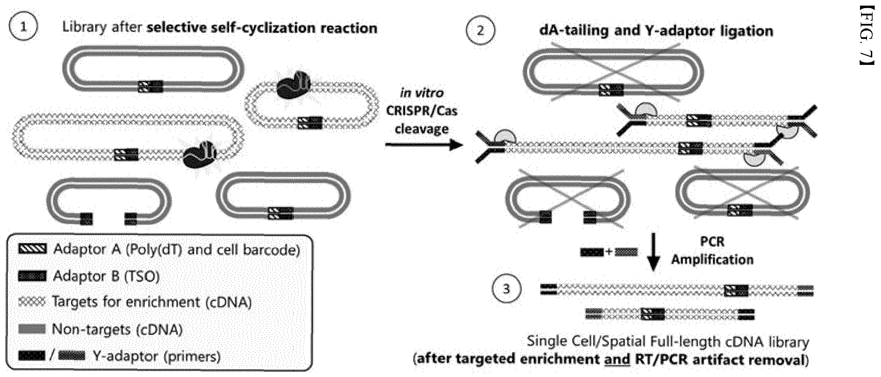

FIG. 7 shows the operating principle of full-length cDNA target selection using the multi-combination assembly reaction of DNA fragments.
FIG. 8 shows an example in which the full-length cDNA target selection method using the multi-combination assembly reaction of DNA fragments is applied.
FIG. 9 shows an example in which the full-length cDNA target selection method using the multi-combination assembly reaction of DNA fragments is applied to a peripheral blood mononuclear cell (PBMC) sample from a patient with relapsed acute myeloid leukemia (AML).

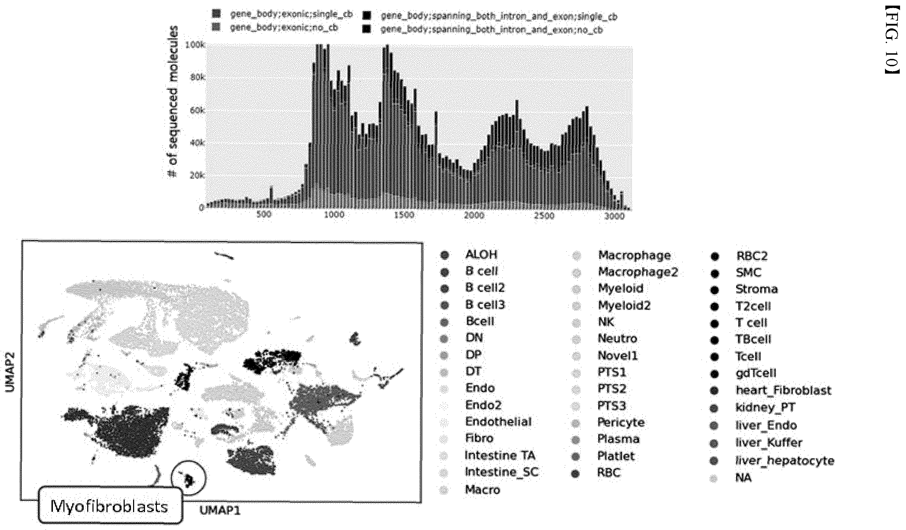

FIG. 10 shows the results of selecting DNA molecules with cell barcodes using the multi-combination assembly reaction of DNA fragments.
FIG. 11 shows an example in which the full-length cDNA target selection method is applied.
FIG. 12 shows the results of generating a single-cell RNA-seq library using SPLiT-Seq technology.
FIG. 13 shows a comparison between results with or without applying the Ouroboros cell barcode selection technology of the present invention.

### [Best Mode]

SPLiT-Seq technology was used to create single-cell RNA-seq libraries from two mouse cell lines and one human cell line. As a result, before applying the Ouroboros cell barcode selection technology of the present invention, the ratio of molecules with cell barcodes was 8.3%, but after applying the Ouroboros cell barcode selection technology, the ratio increased significantly to 85.1%, more than 10 times higher.

### [Mode for Invention]

Hereinafter, the present disclosure will be described in detail with reference to the following examples. However, the following examples are merely illustrative of the present disclosure, and the content of the present disclosure is not limited by the following examples.

### Example

### [Example 1]

### 1. Multi-combination assembly reaction protocol of DNA fragments

### Step 1 - Preparation and purification of single-cell cDNA library

The 10X Genomics Single Cell 3' Expression Kit or other single-cell RNA-seq library preparation methods can be used.

### Step 2 - Selection of DNA molecules with cell barcodes

① Perform PCR using primers that contain part of R1 plus an additional 22 bp, and part of TSO plus an additional 22 bp, in order to add a 22 bp multi-combination assembly adapter necessary for self-circularization assembly reaction to the ends of the molecules containing R1 and TSO sequences, using the program indicated below.

**[Table 1]**

| Lid Temperature | Reaction Volume | Run Time |
|---|---|---|
| 105°C | 50 µl | 20 min |
| Step | Temperature | Duration |
| 1 | 98°C | 00:03:00 |
| 2 | 98°C | 00:00:15 |
| 3 | 63°C | 00:00:20 |
| 4 | 72°C | 00:03:00 |
| 5 | Go to Step 2 four times (total 5 cycles of Step2-5) | |
| 6 | 72°C | 00:01:00 |
| 7 | 4°C | Hold |

② Assemble the self-ligation reaction mixture according to the following table and program. Before setting up the reaction, transfer the NEBuilder HiFi DNA Assembly Master Mix to ice, and gently tap the tube with a finger to mix before use.

**[Table 2]**

| Component | Amount (Final Concentration) |
|---|---|
| 2X NEBuilder HiFi DNA Assembly Master Mix | 5 µl |
| DNA | 10 ~ 100 ng (1 ~ 10ng/µl) |
| Nuclease-free Water | to 10 µl |
| Total Reaction Volume | 10 µl |

**[Table 3]**

| **Lid Temperature** | **Reaction Volume** | **Run Time** |
|---|---|---|
| 105°C | 10 µl | 60 min |
| Step | Temperature | Duration |
| 1 | 50°C | 01:00:00 |
| 2 | 4°C | Hold |

③ On ice, add 1 µL of Exonuclease III, 1 µL of Lambda Exonuclease, and 1 µL of Exonuclease I to the reaction mixture, and remove linear DNA molecules using the following program.

**[Table 4]**

| **Lid Temperature** | **Reaction Volume** | **Run Time** |
|---|---|---|
| 105°C | 12 µl | 30 min |
| Step | Temperature | Duration |
| 1 | 37°C | 00:20:00 |
| 2 | 70°C | 00:10:00 |
| 3 | 4°C | Hold |

④ After the degradation process, add 14 µL of SPRI beads to purify the circular DNA, and elute the purified circular DNA with 10 µL of elution buffer. To confirm the amount of circular DNA molecules, analyze using the Invitrogen Qubit 4 Fluorometer (using 1 µL).
⑤ In a new tube, add the reagents in the following order to assemble Cas9-gRNA ribonucleoproteins targeting the self-ligation junction, and incubate at room temperature for 10 minutes.

**[Table 5]**

| **Component** | **Amount (Final Concentration)** |
|---|---|
| Nuclease-free water | 7 µl |
| NEBuffer r3.1 (10x) | 2 µl |
| 300 nM sgRNA * (10ng/ul) | 9 µl (90 nM final, total 90ng sgRNA) |
| 1 µM Cas9 Nuclease (160ng/ul) ** | 2 µl (~66 nM final) |
| Reaction volume | 20 µl |
| incubate for 10 minutes at room temperature | |

⑥ Set up the following reaction at room temperature and linearize all circular DNA molecules using the following program.

**[Table 6]**

| **Component** | **Amount (Final Concentration)** |
|---|---|
| Purified circular DNA | 9 µl |
| NEBuffer r3.1 (10x) | 1 µl |
| Assembled Cas9-gRNA ribonucleoproteins | 20 µl |
| Total Reaction volume | 30 µl |

**[Table 7]**

| **Lid Temperature** | **Reaction Volume** | **Run Time** |
|---|---|---|
| 105°C | 30 µl | 25 min |
| Step | Temperature | Duration |
| 1 | 37°C | 00:25:00 |
| 2 | 4°C | Hold |

⑦ Add 1 µL of Proteinase K to the reaction mixture, gently mix, and use a centrifuge to collect the solution at the bottom of the tube.
⑧ Incubate at room temperature for 10 minutes.
⑨ Add 37 µl of SPRI beads for DNA clean-up, and elute the purified DNA with 11 µl of elution buffer. Analyze the amount of circular DNA molecules using a Qubit (using 1 µl).
⑩ Perform PCR with 50 µL volume using primers for part of R1 and part of TSO sequences, using Q5 DNA polymerase or KAPA HiFi HotStart ReadyMix, and amplify full-length cDNA using the program shown below.

**[Table 8]**

| **Lid Temperature** | **Reaction Volume** | **Run Time** |
|---|---|---|
| 105°C | 50 µl | 35 min |

| Step | Temperature | Duration |
|---|---|---|
| 1 | 98°C | 00:03:00 |
| 2 | 98°C | 00:00:15 |
| 3 | 63°C | 00:00:20 |
| 4 | 72°C | 00:03:00 |
| 5 | Go to Step 2 seven times (total 8 cycles of Step2-5) | |
| 6 | 72°C | 00:01:00 |
| 7 | 4°C | Hold |

⑪ Perform DNA purification using SPRI beads, and analyze the sample using Qubit or Bioanalyzer to check the quantity and quality of the DNA contained in the sample.
It is a process of amplifying linearized full-length molecules (molecules that have both a 'head' and a 'tail') through a PCR reaction.

### Step 3 - Long-read sequencing

Analyze the full-length molecules derived from single cells using Nanopore/PacBio long-read sequencing technology.

Analysis of the multi-combination assembly reaction protocol of DNA fragments

This is the process of the multi-combination reaction of DNA fragments. Only molecules with both 'head' (R1 and cell barcode, representing the 3' end of mRNA) and 'tail' (TSO, representing the 5' end of mRNA) are assembled into circular form, while the remaining molecules are left unassembled. After the multi-combination reaction of DNA fragments is complete, molecules that are not assembled into circular form are removed. During this process, linear molecules are removed by Exonuclease. Technically, using a combination of various Exonucleases is effective. Once the removal of the linear molecules that did not assemble into circular form is completed, the circular molecules are re-linearized using CRISPR/Cas9 Endonuclease, which precisely recognizes the multi-combination adapter sequences of the DNA fragments and performs linearization. Other Endonucleases, such as restriction enzymes, may also be used.

### 2. Operating Principle of the Selection Method Using the Multi-Combination Assembly Reaction of DNA Fragments

The multi-combination assembly reaction of DNA fragments operates based on the characteristic of self-circularization. The operating principle begins with self-circularization, where the DNA form contains both the cell barcode and Read1 (R1) primer added to the 3' end of the mRNA, as well as the Template-Switching-Oligo (TSO) primer added to the 5' end. Next, circular DNA molecules are selected, while those that do not circularize are removed by exonucleases. Afterward, the linearization step is performed, and the single-cell full-length cDNA library is selected.

### 3. Results of the Multi-Combination Assembly Reaction of DNA Fragments

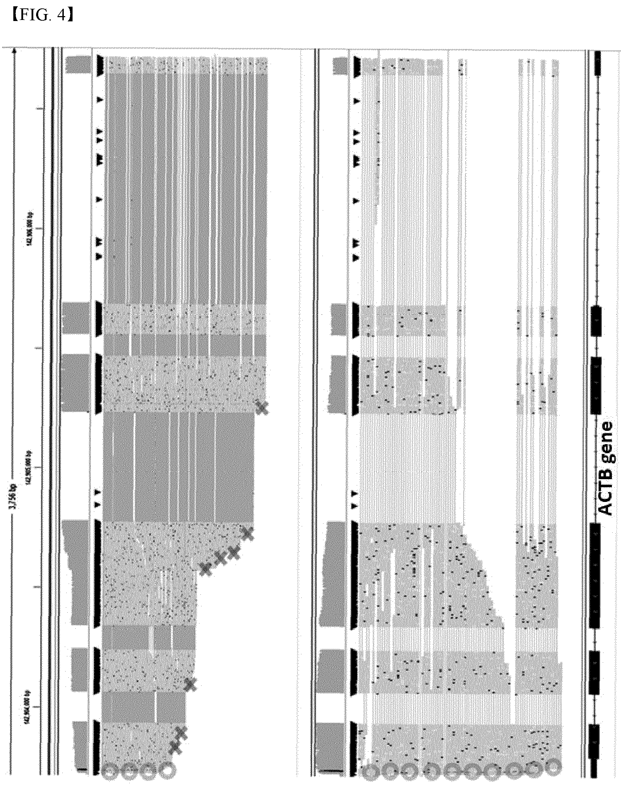

Using the multi-combination assembly reaction of DNA fragments, DNA molecules with cell barcodes are selected. The multi-combination assembly reaction of DNA fragments was used for cell separation from the mouse heart. First, single cells were isolated from the mouse heart. Then, a 10X single-cell transcriptome library was created, and the process of selecting DNA molecules with cell barcodes using the multi-combination assembly reaction of DNA fragments was performed, ultimately leading to the selection of the target molecules. In the selection process of mouse heart single cells, it was confirmed that molecules with correctly attached cell barcodes were effectively selected during the multi-combination assembly reaction (Fig. 4).

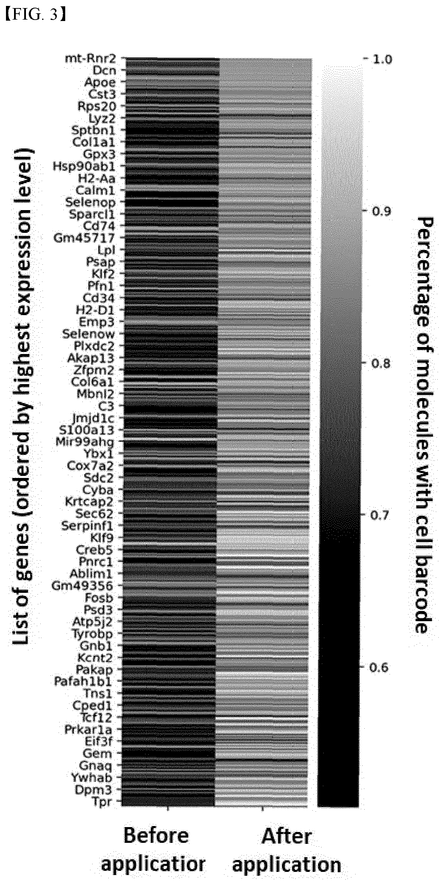

As a result of selection, the ratio of molecules with cell barcodes in various genes was improved from below 50% to above 90% (Fig. 3). The same effectiveness was confirmed in five other tissues from the mouse, excluding the heart.

### 4. Technical Validation

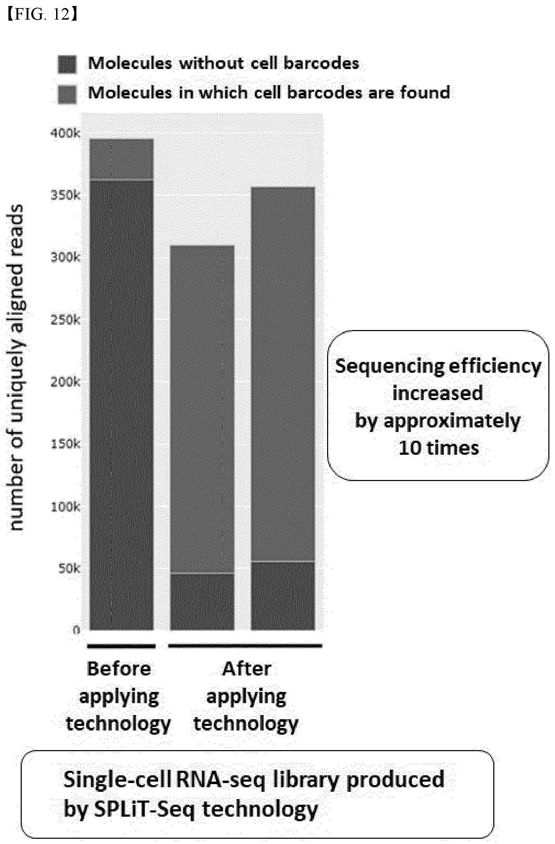

The results of applying the SPLiT-Seq technology to single-cell RNA-Seq libraries created using this technology are as follows. SPLiT-Seq technology was used to create single-cell RNA-seq libraries from two mouse cell lines and one human cell line. As a result, before applying the Ouroboros cell barcode selection technology of the present invention, the ratio of molecules with cell barcodes was 8.3%, but after applying the Ouroboros cell barcode selection technology, the ratio increased significantly to 85.1%, more than 10 times higher (Fig. 12).

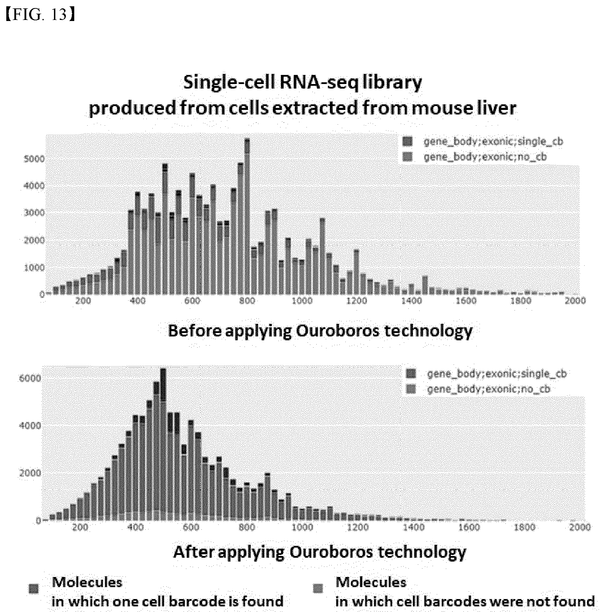

Using the 10X Genomics technology (3' Gene Expression Kit, v3), a single-cell RNA-seq library was created from cells isolated from mouse liver tissue. Before applying the Ouroboros cell barcode selection technology of the present invention, the ratio of molecules with cell barcodes was 19.1%, but after applying the Ouroboros cell barcode selection technology, the ratio increased to 91.1%, approximately 4.8 times higher (Fig. 13).

### [Example 2]

### 1. Multi-Combination Assembly Reaction Protocol of DNA Fragments (Selective DNA Molecule Removal)

### Step 1 - Preparation and purification of single-cell cDNA library

The 10X Genomics Single Cell 3' Expression Kit or other single-cell RNA-seq library preparation methods can be used.

### Step 2 - Selection of DNA molecules with cell barcodes and Selective DNA Molecule Removal

① Perform PCR using primers that contain part of R1 plus an additional 22 bp, and part of TSO plus an additional 22 bp, in order to add a 22 bp multi-combination assembly adapter necessary for self-circularization assembly reaction to the ends of the molecules containing R1 and TSO sequences, using the program indicated below.

**[Table 9]**

| **Lid Temperature** | **Reaction Volume** | **Run Time** |
|---|---|---|
| 105°C | 50 µl | 20 min |

| Step | Temperature | Duration |
|---|---|---|
| 1 | 98°C | 00:03:00 |
| 2 | 98°C | 00:00:15 |
| 3 | 63°C | 00:00:20 |
| 4 | 72°C | 00:03:00 |
| 5 | Go to Step 2 four times (total 5 cycles of Step2-5) | |
| 6 | 72°C | 00:01:00 |
| 7 | 4°C | Hold |

② Assemble the self-ligation reaction mixture according to the following table and program. Before setting up the reaction, transfer the NEBuilder HiFi DNA Assembly Master Mix to ice, and gently tap the tube with a finger to mix before use.

**[Table 10]**

| **Component** | **Amount (Final Concentration)** |
|---|---|
| 2X NEBuilder HiFi DNA Assembly Master Mix | 5 µl |
| DNA | 10 ~ 100 ng (1 ~ 10ng/µl) |
| Nuclease-free Water | to 10 µl |
| Total Reaction Volume | 10 µl |

**[Table 11]**

| **Lid Temperature** | **Reaction Volume** | **Run Time** |
|---|---|---|
| 105°C | 10 µl | 60 min |

| Step | Temperature | Duration |
|---|---|---|
| 1 | 50°C | 01:00:00 |
| 2 | 4°C | Hold |

③ In a new tube, add the reagents in the following order to assemble Cas9-gRNA ribonucleoproteins, and incubate at room temperature for 10 minutes.

**[Table 12]**

| **Component** | **Amount (Final Concentration)** |
|---|---|
| Nuclease-free water | 7 µl |
| NEBuffer r3.1 (10x) | 2 µl |
| 1.5 µM sgRNA (sgRNA molecules that recognize the targets to be removed. In this implementation example, sgRNA molecules that recognize MT DNA and rRNA are used.) (50ng/ul) | 9.5 µl (~475 nM final, total 475ng sgRNA) |
| 20 µM Cas9 Nuclease, S. pyogenes | 0.5 µl (~300 nM final) |
| Reaction volume | 20 µl |
| incubate for 10 minutes at room temperature | |

④ Set up the following reaction at room temperature and linearize the undesired circular DNA molecules using the following program.

**[Table 13]**

| **Component** | **Amount (Final Concentration)** |
|---|---|
| Gibson reaction mixture (DNA) | 10 µl |
| Assembled Cas9-gRNA ribonucleoproteins | 20 µl |
| Total Reaction volume | 30 µl |

**[Table 14]**

| **Lid Temperature** | **Reaction Volume** | **Run Time** |
|---|---|---|
| 105°C | 30 µl | 30 min |

| Step | Temperature | Duration |
|---|---|---|
| 1 | 37°C | 00:30:00 |
| 2 | 4°C | Hold |

⑤ Add 1 µl of Thermolabile Proteinase K to the reaction mixture. Mix gently and pulse spin in a centrifuge. Incubate the reaction mixture using the following program.

**[Table 15]**

| **Lid Temperature** | **Reaction Volume** | **Run Time** |
|---|---|---|
| 105°C | 31 µl | 25 min |

| Step | Temperature | Duration |
|---|---|---|
| 1 | 37°C | 00:15:00 |
| 2 | 55°C | 00:10:00 |
| 3 | 4°C | Hold |

⑥ Add 1 µl of Exonuclease III, 1 µl of Lambda Exonuclease, and 1 µl of Exonuclease I to the reaction mixture on ice. Remove linear DNA molecules using the following program.

**[Table 16]**

| **Lid Temperature** | **Reaction Volume** | **Run Time** |
|---|---|---|
| 105°C | 33 µl | 30 min |
| Step | Temperature | Duration |
| 1 | 37°C | 00:20:00 |
| 2 | 70°C | 00:10:00 |
| 3 | 4°C | Hold |

⑦ After the degradation reaction of the linear DNA is complete, add 14 µl of SPRI beads to purify the circular DNA, and elute the purified DNA with 10 µl of elution buffer. Analyze the amount of circular DNA molecules using a Qubit (using 1 µl).
⑧ In a new tube, add the reagents in the following order to assemble Cas9-gRNA ribonucleoproteins targeting the self-ligation junction, and incubate at room temperature for 10 minutes.

**[Table 17]**

| **Component** | **Amount (Final Concentration)** |
|---|---|
| Nuclease-free water | 7 µl |
| NEBuffer r3.1 (10x) | 2 µl |
| 300 nM sgRNA * (10ng/ul) | 9 µl (90 nM final, total 90ng sgRNA) |
| 1 µM Cas9 Nuclease (160ng/ul) ** | 2 µl (~66 nM final) |
| Reaction volume | 20 µl |
| incubate for 10 minutes at room temperature | |

⑨ Set up the following reaction at room temperature and linearize circular DNA molecules using the following program.

**[Table 18]**

| **Component** | **Amount (Final Concentration)** |
|---|---|
| Purified circular DNA | 9 µl |
| NEBuffer r3.1 (10x) | 1 µl |
| Assembled Cas9-gRNA ribonucleoproteins | 20 µl |
| Total Reaction volume | 30 µl |

**[Table 19]**

| **Lid Temperature** | **Reaction Volume** | **Run Time** |
|---|---|---|
| 105°C | 30 µl | 25 min |
| Step | Temperature | Duration |
| 1 | 37°C | 00:25:00 |
| 2 | 4°C | Hold |

⑩ Add 1 µL of Proteinase K to the reaction mixture, gently mix, and pulse-spin using a centrifuge.
⑪ Incubate at room temperature for 10 minutes.
⑫ After the digestion reaction, add 37 µl of SPRI beads for DNA clean-up, and elute the purified DNA with 11 µl of elution buffer. Analyze the amount of circular DNA molecules using a Qubit (using 1 µl).
⑬ Perform PCR with 50 µL volume using primers for part of R1 and part of TSO sequences, using Q5 DNA polymerase or KAPA HiFi HotStart ReadyMix, and amplify full-length cDNA using the program shown below.

**[Table 20]**

| **Lid Temperature** | **Reaction Volume** | **Run Time** |
|---|---|---|
| 105°C | 50 µl | 35 min |
| Step | Temperature | Duration |
| 1 | 98°C | 00:03:00 |
| 2 | 98°C | 00:00:15 |
| 3 | 63°C | 00:00:20 |
| 4 | 72°C | 00:03:00 |
| 5 | Go to Step 2 seven times (total 8 cycles of Step2-5) | |
| 6 | 72°C | 00:01:00 |
| 7 | 4°C | Hold |

⑭ Perform DNA purification using SPRI beads, and analyze the sample using Qubit or Bioanalyzer to check the quantity and quality of the DNA contained in the sample.

Above ⑬ to ⑭ are a process of amplifying linearized full-length molecules (molecules that have both a 'head' and a 'tail') through a PCR reaction.

### Step 3 - Long-read sequencing (Nanopore/PacBio)

Analyze the full-length molecules derived from single cells using Nanopore/PacBio long-read sequencing technology.

Analysis of the multi-combination assembly reaction protocol of DNA fragments (Selective DNA Molecule Removal)

This is the process of the multi-combination reaction of DNA fragments. Only molecules with both 'head' (R1 and cell barcode, representing the 3' end of mRNA) and 'tail' (TSO, representing the 5' end of mRNA) are assembled into circular form, while the remaining molecules are left unassembled. The process of removing unwanted DNA using CRISPR/Cas9 is highly accurate, as the CRISPR/Cas9 system allows for the precise removal of only the targeted molecules. One of the advantages of this method is the ability to easily remove hundreds of targets simultaneously.

After the completion of the multi-linking assembly reaction of DNA fragments, the process of removing molecules that were not assembled into a circular form, as well as linearized DNA molecules among those that were originally circular, takes place. The linear molecules are removed by exonucleases. Technically, using a combination of different exonucleases is effective. Furthermore, after the removal of the linear molecules that were not assembled into a circular form, the process of re-converting the circular molecules back into a linear form is carried out using CRISPR/Cas9 endonuclease, which precisely recognizes the multi-linking adapter sequences of the DNA fragments and proceeds with the linearization. Additionally, other endonucleases, such as restriction enzymes, can also be used.

### 2. Mechanism and Application of Multi-Linking Assembly Reaction of DNA Fragments (Selective DNA Molecule Removal)

After the multi-linking assembly reaction of DNA fragments, the process of removing selective DNA molecules begins in the full-length DNA library. The process for removing selective DNA molecules is as follows:
1) Full-length DNA library self-assembled into circular DNA
2) Target DNA molecules containing a specific DNA sequence (from one to thousands) with CRISPR/Cas9 nuclease (selectively converting unwanted DNA targets into linear DNA)
3) Circular DNA purification, selection of full-length DNA molecules with unwanted DNA molecules removed

Through these steps, the selective DNA molecules are removed, and the remaining circular DNA is subjected to a linearization process. Subsequently, the analysis proceeds with the single-cell full-length DNA library.

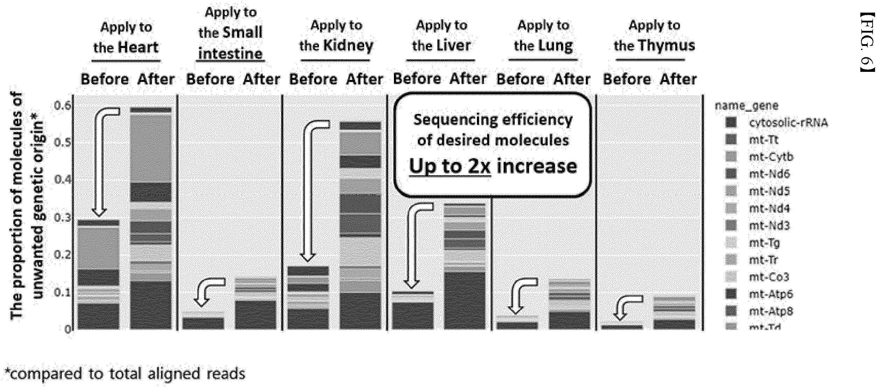

CRISPR/Cas9 recognizes the target DNA sequence in the unwanted DNA molecules, converting them into linear DNA, and ultimately purifies the remaining circular DNA. After this, the linearization is achieved and the linear molecules are amplified via PCR for further analysis. The results of the selective DNA molecule removal process are shown in Figure 6. In this case, 26 types of sgRNA targeting ribosomal and mitochondrial genome-derived cDNA were used, and increasing the number of sgRNAs will enable more efficient removal. The selective DNA molecule removal method was applied to mouse tissue, and it was confirmed that the sequencing efficiency of the desired molecules increased by up to two times.

### 3. Technical Validation

Using 26 target sequences that target mitochondrial and ribosomal RNA-derived cDNA, mitochondrial and ribosomal RNA-derived cDNA were removed from the single-cell RNA-Seq library. The analysis was conducted by preparing single-cell RNA-seq libraries from single cells isolated from various mouse tissues using the 10X Genomics technology (3' Gene Expression Kit, v3). As a result, before applying the Ouroboros cell barcode selection and molecular target removal technology, the ratio of mitochondrial and ribosomal RNA-derived cDNA molecules in the library produced from kidney tissue was 56%. However, after applying the Ouroboros cell barcode selection and molecular target removal technology, the ratio of mitochondrial and ribosomal RNA-derived cDNA molecules in the kidney tissue library was reduced to 17%. The sequencing efficiency of the desired molecules (non-mitochondrial and non-ribosomal RNA-derived cDNA molecules) increased approximately two-fold, from 44% to 83% (Figure 6).

### [Example 31

### 1. Multi-combination assembly reaction protocol of DNA fragments (DNA Target Selection)

### Step 1 - Preparation and purification of single-cell cDNA library

The 10X Genomics Single Cell 3' Expression Kit or other single-cell RNA-seq library preparation methods can be used.

### Step 2 - Selection of DNA molecules with cell barcodes

① Perform PCR using primers that contain part of R1 plus an additional 22 bp, and part of TSO plus an additional 22 bp, in order to add a 22 bp multi-combination assembly adapter necessary for self-circularization assembly reaction to the ends of the molecules containing R1 and TSO sequences, using the program indicated below.

**[Table 21]**

| **Lid Temperature** | **Reaction Volume** | **Run Time** |
|---|---|---|
| 105°C | 50 µl | 20 min |
| Step | Temperature | Duration |
| 1 | 98°C | 00:03:00 |
| 2 | 98°C | 00:00:15 |
| 3 | 63°C | 00:00:20 |
| 4 | 72°C | 00:03:00 |
| 5 | Go to Step 2 four times (total 5 cycles of Step2-5) | |
| 6 | 72°C | 00:01:00 |
| 7 | 4°C | Hold |

② Assemble the self-ligation reaction mixture according to the following table and program. Before setting up the reaction, transfer the NEBuilder HiFi DNA Assembly Master Mix to ice, and gently tap the tube with a finger to mix before use.

**[Table 22]**

| **Component** | **Amount (Final Concentration)** |
|---|---|
| 2X NEBuilder HiFi DNA Assembly Master Mix | 5 µl |
| DNA | 10 ~ 100 ng (1 ~ 10ng/µl) |
| Nuclease-free Water | to 10 µl |
| Total Reaction Volume | 10 µl |

**[Table 23]**

| **Lid Temperature** | **Reaction Volume** | **Run Time** |
|---|---|---|
| 105°C | 10 µl | 60 min |
| Step | Temperature | Duration |
| 1 | 50°C | 01:00:00 |
| 2 | 4°C | Hold |

③ On ice, add 1 µL of Exonuclease III, 1 µL of Lambda Exonuclease, and 1 µL of Exonuclease I to the reaction mixture, and remove linear DNA molecules using the following program.

**[Table 24]**

| **Lid Temperature** | **Reaction Volume** | **Run Time** |
|---|---|---|
| 105°C | 12 µl | 30 min |
| Step | Temperature | Duration |
| 1 | 37°C | 00:20:00 |
| 2 | 70°C | 00:10:00 |
| 3 | 4°C | Hold |

④ After the degradation process, add 14 µL of SPRI beads to clean up, and elute the purified circular DNA with 10 µL of elution buffer. And to confirm the amount of circular DNA molecules, analyze using the Invitrogen Qubit 4 Fluorometer (using 1 µL).
⑤ In a new tube, add the reagents in the following order to assemble Cas9-gRNA ribonucleoproteins targeting the self-ligation junction, and incubate at room temperature for 10 minutes.

**[Table 25]**

| **Component** | **Amount (Final Concentration)** |
|---|---|
| Nuclease-free water | 7 µl |
| NEBuffer r3.1 (10x) | 2 µl |
| 300 nM sgRNA * (10ng/ul) | 9 µl (90 nM final, total 90ng sgRNA) |
| 1 µM Cas9 Nuclease (160ng/ul) ** | 2 µl (~66 nM final) |
| Reaction volume | 20 µl |
| incubate for 10 minutes at room temperature | |

⑥ Set up the following reaction at room temperature and linearize all circular DNA molecules using the following program.

**[Table 26]**

| **Component** | **Amount (Final Concentration)** |
|---|---|
| Purified circular DNA | 9 µl |
| NEBuffer r3.1 (10x) | 1 µl |
| Assembled Cas9-gRNA ribonucleoproteins | 20 µl |
| Total Reaction volume | 30 µl |

**[Table 27]**

| **Lid Temperature** | **Reaction Volume** | **Run Time** |
|---|---|---|
| 105°C | 30 µl | 25 min |
| Step | Temperature | Duration |
| 1 | 37°C | 00:25:00 |
| 2 | 4°C | Hold |

⑦ Add 1 µL of Proteinase K to the reaction mixture, gently mix, and pulse-spin using a centrifuge.
⑧ Incubate at room temperature for 10 minutes.
⑨ After the digestion reaction, add 37 µl of SPRI beads for DNA clean-up, and elute the purified DNA with 11 µl of elution buffer. Analyze the amount of circular DNA molecules using a Qubit (using 1 µl).
⑩ Set up the following dA-tailing (End-Prep) reaction at room temperature, and incubate at 20°C for 20 minutes, then at 65°C for 10 minutes.

**[Table 28]**

| **Component** | **Amount (Final Concentration)** |
|---|---|
| Cas9-Digested target DNA | 10 µl |
| Ultra II End-prep reaction buffer | 1.75 µl |
| Ultra II End-prep enzyme mix | 0.75 µl |
| Nuclease-free water | 2.5 µl (to 15 µl) |
| Total Reaction volume | 15 µl |

⑪ For clean purification, add 20µl of SPRI beads and elute the purified DNA with 11µl of elution buffer.
⑫ Set up the following reaction at room temperature and incubate for 30 minutes at room temperature.

**[Table 29]**

| **Component** | **Amount (Final Concentration)** |
|---|---|
| End-Prepped DNA | 10 µl |
| Hybridized Y-adaptor (T-overhang) (0.5 µM)* (diluted in nuclease-free water) | 1 µl |
| Blunt/TA Ligase Master Mix | 14 µl |
| Total Reaction volume | 25 µl |

⑬ For clean purification, add 20µl of SPRI beads and elute the purified DNA with 11µl of elution buffer.
Above ⑫ to ⑬ are a process of attaching a Y-adaptor to the linear target DNA (End-Prepped DNA).
⑭ Using forward and reverse primers for the Y-adaptor, perform a 50µl PCR with Q5 DNA polymerase or KAPA HiFi HotStart ReadyMix, following the program outlined below, to selectively amplify the full-length cDNA targets.

**[Table 30]**

| **Lid Temperature** | **Reaction Volume** | **Run Time** |
|---|---|---|
| 105°C | 50 µl | 60 min |
| Step | Temperature | Duration |
| 1 | 98°C | 00:03:00 |
| 2 | 98°C | 00:00:15 |
| 3 | 61°C | 00:00:20 |
| 4 | 72°C | 00:03:00 |
| 5 | Go to Step 2 9~11 times (total 10~12 cycles of Step2-5) | |
| 6 | 72°C | 00:01:00 |
| 7 | 4°C | Hold |

⑮ Clean up the SPRI beads, and analyze the sample using Qubit or Bioanalyzer to check the quantity and quality of the DNA contained in the sample.

Above ⑭ to ⑮ are a process of amplifying the linear DNA targets with the appropriate primers for the Y-adaptor used in the ligation. During this amplification process, the unselected circular DNA is removed.

### Step 3 - Long-read sequencing (Nanopore/PacBio)

Analyze the full-length molecules derived from single cells using Nanopore/PacBio long-read sequencing technology.

Analysis of the multi-combination assembly reaction protocol of DNA fragments (DNA target selection)

This is the process of the multi-combination reaction of DNA fragments. Only molecules with both 'head' (R1 and cell barcode, representing the 3' end of mRNA) and 'tail' (TSO, representing the 5' end of mRNA) are assembled into circular form, while the remaining molecules are left unassembled. After the multi-combination reaction of DNA fragments is complete, molecules that are not assembled into circular form are removed. During this process, linear molecules are removed by Exonuclease. Technically, using a combination of various Exonucleases is important. Once the removal of the linear molecules that did not assemble into circular form is completed, the circular molecules are re-linearized using CRISPR/Cas9 Endonuclease, which precisely recognizes the multi-combination adapter sequences of the DNA fragments and performs linearization. Other Endonucleases, such as restriction enzymes, may also be used.

The protocol for the multi-fragment assembly reaction of DNA targets is a process where CRISPR/Cas9 is used to select the desired DNA targets without loss of information. By utilizing the CRISPR/Cas9 system, it is possible to accurately select only the desired molecules, and it has the advantage of being able to efficiently select hundreds of targets simultaneously. When assuming the same number of targets, this method is simpler and more economical compared to traditional technologies such as RNA-hybrid capture. Technically, before performing the Y-adaptor ligation reaction, it is essential to use the CRISPR/Cas9 system to add A-tails to the target DNAs that have been converted to linear form. This step is crucial to reduce adaptor-dimer formation and increase the efficiency of the reaction.

### 2. Mechanism and Application of Multi-Linking Assembly Reaction of DNA Fragments (Selective DNA Molecule Removal)

After the multi-linking assembly reaction of DNA fragments, the process of removing selective DNA molecules begins in the full-length DNA library in a circular form. The process for selection target DNA molecules is as follows:
1) Full-length DNA library self-assembled into circular DNA
2) Target DNA molecules derived from genes/cells of interest (from one to thousands) with CRISPR/Cas9 nuclease (selectively converting circular DNA molecules containing target DNA sequences into linear DNA)
3) Y-adaptor ligation only to linear DNA, target selection through amplification process

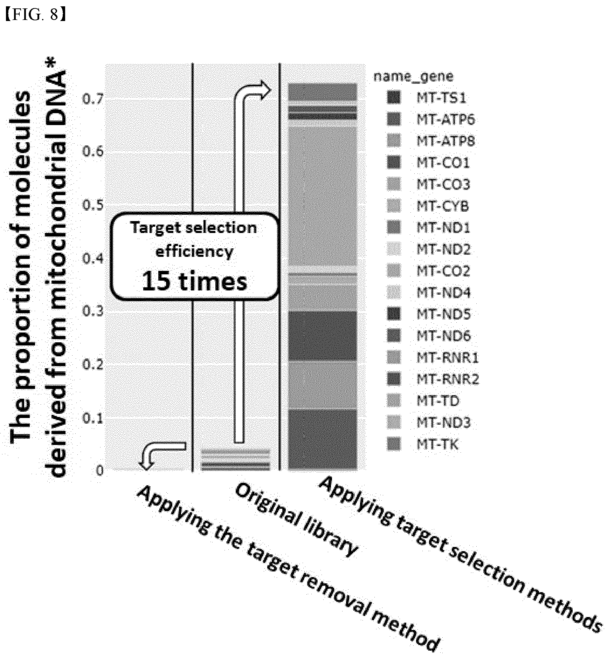

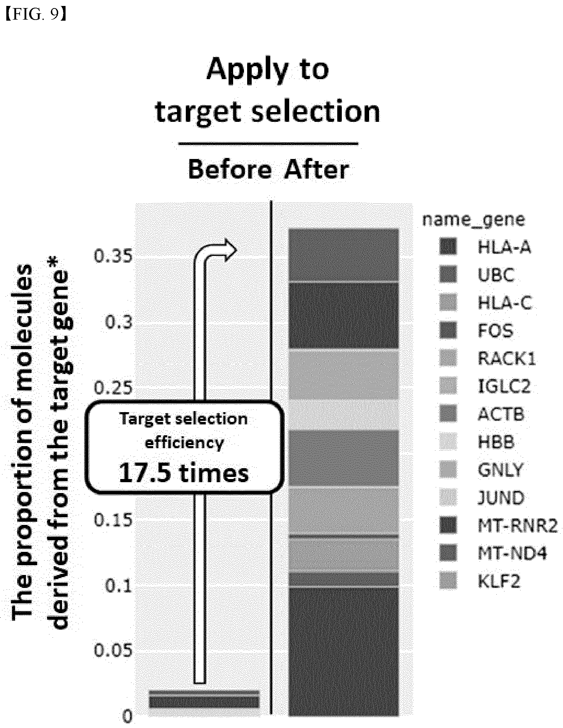

The results of cDNA target selection are shown in Figure 8. The process was carried out using a mouse tissue sample with the lowest amount of mitochondrial genome-derived cDNA. cDNA derived from 11 genes in the mitochondrial genome was targeted, and after target selection, 72% of the sequenced bases were identified as belonging to the target cDNA. The target selection efficiency was found to be 15 times higher compared to the original library. Additionally, single-cell isolation was performed on a peripheral blood mononuclear cell (PBMC) sample from a cancer patient, and full-length cDNA target selection was carried out. A 10X single-cell transcriptome library was generated from the isolated single cells, and 22 sgRNAs targeting regions with single nucleotide variants (SNVs) were used for the target selection. As a result, the target selection efficiency increased by 17.5 times (Figure 9).

### 3. Technical Validation

The results of selectively targeting mitochondrial genome-derived cDNA in a single-cell RNA-Seq library using 11 types of mitochondrial genome-derived cDNA are as follows. A single-cell RNA-seq library was generated using thymus tissue from a mouse, isolated with the 10X Genomics 3' Gene Expression Kit (v3). Before applying the Ouroboros cell barcode selection and molecular target selection technology of the present invention, the proportion of mitochondrial genome-derived cDNA molecules in the library was 4.2%. After applying the Ouroboros technology, the proportion increased to 72%, indicating that the sequencing efficiency of the desired molecules (mitochondrial genome-derived cDNA) increased by approximately 17-fold (Figure 8).

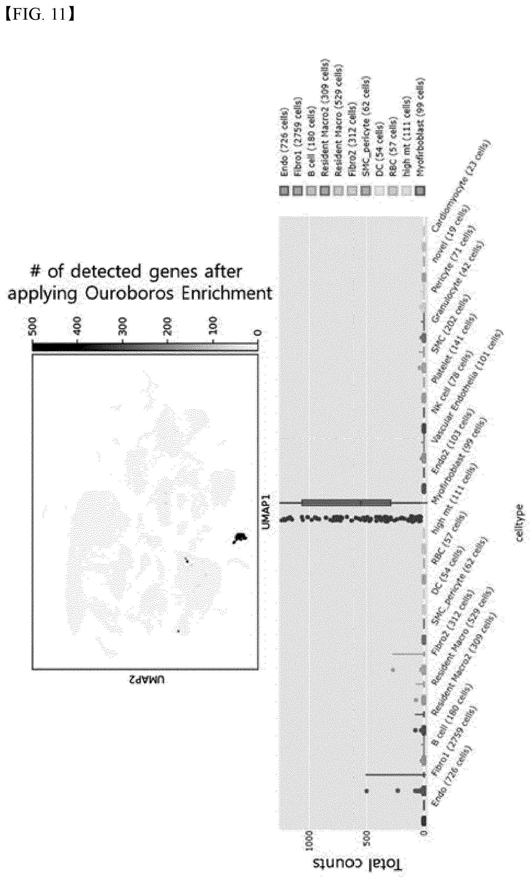

The following are the results of selectively isolating only the cDNA molecules derived from 99 fibroblast cells identified within a single-cell library containing cDNA from 6,500 single cells. A single-cell RNA-seq library was prepared using kidney tissue from a mother mouse, processed with the 10X Genomics 3' Gene Expression Kit (v3). Through sequencing and bioinformatics analysis, the cell barcodes of the 99 fibroblast cells were identified. DNA oligos specific to these 99 cell barcodes were designed using IDT's oPool service. CRISPR/Cas9 sgRNAs targeting the 99 cell barcodes were then generated using these DNA oligos. Application of the Ouroboros cell barcode selection and molecular target selection technology confirmed that only the cDNA derived from the 99 fibroblast cells was accurately selected (Figure 11).

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

### [Industrial Applicability]

The present invention is a development study to overcome the low quality and high cost of multiome full-length sequencing, including transcriptomes of single cells, and the limitations of cellular heterogeneity studies. By using an assembly method that allows the combination of multiple DNA fragments, it significantly improves the efficiency and error rate of full-length sequencing, and enables multiome full-length sequencing of single cells. It is expected to be widely used for expression analysis of gene isoforms, mutation detection, or cancer cell-specific multiome analysis.

## Claims

1. A method for full-length multiome sequencing analysis of a single cell, comprising:
(a) attaching a multi-ligation assembly adapter of DNA fragments to a multiome library prepared from a single cell;
(b) selectively circularizing DNA molecules in the library through a multi-ligation assembly reaction of DNA fragments to select DNA molecules within the library; and
(c) removing DNA fragments other than the circular DNA using an exonuclease.

2. The method according to claim 1,
further comprising after step (c):
(d) linearizing the circular DNA in the library.

3. The method according to claim 1,
wherein the multiome is a transcriptome, spatial transcriptome, genome, spatial genome, proteome, epigenome, or spatial epigenome.

4. The method according to claim 1,
wherein the multiome library comprises different adapters (forward and reverse adapters) ligated to the 3' and 5' ends, respectively.

5. The method according to claim 1,
wherein in step (a), two different adapters are assembled to form circular DNA.

6. The method according to claim 2,
wherein the linearization in step (d) is performed using a CRISPR/Cas nuclease, restriction enzyme, or transcription activator-like effector nuclease (TALEN).

7. The method according to claim 6,
wherein the CRISPR/Cas nuclease is selected from the group consisting of CRISPR/Cas9, CRISPR/Cas12, CRISPR/Cas13, and CRISPR/Cas14.

8. A method for sequencing analysis after selective removal of DNA molecules from a full-length multiome library of a single cell, comprising:
(a) attaching a multi-ligation assembly adapter of DNA fragments to a multiome library prepared from a single cell;
(b) generating circular DNA within the library through a multi-ligation assembly reaction of DNA fragments;
(c) selectively linearizing circular DNA containing target sequences to be removed using a CRISPR/Cas nuclease; and
(d) removing linear DNA, which is not circular DNA, using an exonuclease.

9. The method according to claim 8,
further comprising after step (d):
(e) linearizing the circular DNA in the library.

10. The method according to claim 8,
wherein the CRISPR/Cas nuclease is selected from the group consisting of CRISPR/Cas9, CRISPR/Cas12, CRISPR/Cas13, and CRISPR/Cas14.

11. The method according to claim 8,
wherein the CRISPR/Cas nuclease is applied only to circular DNA.

12. The method according to claim 8,
wherein the linear DNA in step (d) is a molecule generated by degradation of selected circular DNA recognized by the CRISPR/Cas nuclease.

13. A method for sequencing analysis after selection of DNA molecules from a full-length multiome library of a single cell, comprising:
(a) preparing a multiome library from a single cell and attaching a multi-ligation assembly adapter of DNA fragments to the library;
(b) generating circular DNA within the library through a multi-ligation assembly reaction of DNA fragments;
(c) removing DNA fragments that are not circular DNA using an exonuclease; and
(d) selectively converting circular DNA containing target sequences to be selected into linear DNA using a CRISPR/Cas nuclease.

14. The method according to claim 13,
further comprising after step (d):
(e) amplifying the linear DNA in the library.

15. The method according to claim 13,
wherein the CRISPR/Cas nuclease is selected from the group consisting of CRISPR/Cas9, CRISPR/Cas12, CRISPR/Cas13, and CRISPR/Cas14.

16. The method according to claim 13,
wherein the linear DNA in step (d) is a molecule generated by degradation of selected circular DNA recognized by the CRISPR/Cas nuclease.

17. The method according to claim 14,
wherein step (e) further comprises a ligation step of a Y-adaptor or a hairpin adaptor.

18. A single-cell full-length multiome sequencing analysis kit using the method of claim 1.

19. A single-cell level gene isoform diagnostic marker analysis kit using the method of claim 1.

20. A single-cell mutation-based cancer targeted therapy candidate analysis kit using the method of claim 1.
